# EUROPEAN PATENT APPLICATION

(11) **EP 4 729 598 A1**
(43) Date of publication of application: **22.04.2026**
(21) Application number: 24822732.4
(22) Date of filing: 13.06.2024
(51) Int. Cl.: C12M 1/33, C12M 1/26, C12M 1/02, C12M 1/34, C12M 1/36, C12M 1/00

(54) **ALKALINE LYSIS DEVICE AND METHOD FOR USING DEVICE TO EXTRACT PLASMIDS**

(30) Priority: 13.06.2023 CN 202310700226
(71) Applicant: Zhenjiang Probio Biotech Co., Ltd., Zhenjiang, Jiangsu 212009 (CN)
(72) Inventor: WEI, Shigang, Zhenjiang, Jiangsu 212009 (CN); SUN, Tao, Zhenjiang, Jiangsu 212009 (CN); ZHU, Jinxiu, Zhenjiang, Jiangsu 212009 (CN); WANG, Peng, Zhenjiang, Jiangsu 212009 (CN); MIAO, Nan, Zhenjiang, Jiangsu 212009 (CN); ZHANG, Guoqiang, Zhenjiang, Jiangsu 212009 (CN); LU, Shengdong, Zhenjiang, Jiangsu 212009 (CN); ZENG, Xuefei, Zhenjiang, Jiangsu 212009 (CN)
(74) Representative: Synergy IP Group AG
(86) International application number: PCT/CN2024/098865
(87) International publication number: WO 2024/255783

(57) **Abstract**

Provided are an alkaline lysis device and a method for using the device to extract plasmids. The alkaline lysis device comprises: a first pipeline, a second pipeline, a third pipeline, a cell lysing reactor, and a neutralization and stationary incubation tank; the first pipeline is used for conveying a bacterial suspension, the second pipeline is used for conveying an alkaline lysate, the third pipeline is used for conveying an acid liquid, the cell lysing reactor is used as a reaction vessel for alkaline lysis of cells, and the neutralization and stationary incubation tank is used for collecting a neutralized feed liquid and carrying out stationary incubation; the first pipeline and the second pipeline intersect and then are connected to the inlet end of the cell lysing reactor; the outlet ends of the third pipeline and the cell lysing reactor intersect and then are connected to the inlet end of the neutralization and stationary incubation tank. The alkaline lysis device can realize online mixing of the bacterial suspension, the alkaline lysate, and the acid liquid at a specific ratio and continuous flow lysis, and the method for using the alkaline lysis device to extract plasmids can obtain high-quality plasmids and is simple, convenient and efficient.

## Description

### Cross Reference to Related Applications

The present application claims priority to Chinese Patent Application No. 202310700226.1, filed on June 13, 2023, which is incorporated herein by reference in its entirety.

### Technical Field

The present invention relates to the field of biotechnology and biological medicine, and in particular to an alkaline lysis device and a method for extracting plasmids using the device.

### Background Art

Plasmids are typically produced from E. coli, and alkaline lysis is used in purification processes for the production of plasmids to extract the plasmids. The alkaline lysis process generally includes three steps: a first step of resuspension of E. coli cells, in which the E. coli cells are usually resuspended in a resuspension buffer by means of manual or magnetic stirring; a second step of alkaline lysis, which is usually carried out by manual stirring, such that the resuspended E. coli cells are mixed with an alkaline lysate and subjected to an action for a certain period of time (typically 3 to 5 minutes), and then the cells are disrupted and release the plasmids and other intracellular materials; and a third step of neutralization, in which an acid liquid is usually added to the previous step and then manually stirred for mixing for the purpose of neutralization, and white insolubles are produced by a displacement reaction and carry denatured chromosomal DNA (deoxyribonucleic acid) as well as denatured proteins and cell debris which are precipitated after aggregation, while plasmid DNA is restored to its native configuration and dissolved in a supernatant. However, the alkaline lysis process generally suffers from the following problems in mass production: 1. The addition of the alkaline solution for lysis is usually done in a stainless steel barrel or a disposable liner bag, and uneven power, shear force, etc. arise in the manual stirring and cause non-uniform mixing, resulting in an excessively high local pH (acid-alkalinity) which results in irreversible deformation of the plasmids. 2. When the volume of the solution reaches hundreds of liters during production, the manual stirring is time-consuming and the shear force is great, thus preventing effective scale-up. 3. For the existing lysis devices disclosed, although mechanical lysis can be achieved, it is mostly limited to laboratory scale, scale-up is impossible, and production in the conditions in accordance with the GMP (Pharmaceutical Production Quality Management Specification) is also impossible.

### Summary

In view of the deficiencies in the prior art, an object of the present invention is to provide an alkaline lysis device and a method for extracting plasmids using the device.

In order to solve the above technical problems, the following technical solutions are employed by the present invention. An alkaline lysis device includes: a first pipeline, a second pipeline, a third pipeline, a cell lysing reactor, and a neutralization and stationary incubation tank; where the first pipeline is configured for conveying a bacterial suspension; the second pipeline is configured for conveying an alkaline lysate; the third pipeline is configured for conveying an acid liquid; the cell lysing reactor is used as a reaction vessel for alkaline lysis of cells; the neutralization and stationary incubation tank is configured for collecting a neutralized feed liquid and carrying out stationary incubation; the first pipeline and the second pipeline intersect and then are connected to an inlet end of the cell lysing reactor; and the third pipeline and an outlet end of the cell lysing reactor intersect and then are connected to an inlet end of the neutralization and stationary incubation tank.

In a specific example, the alkaline lysis device includes: a first static mixer and a second static mixer; where the first static mixer is disposed in a pipeline downstream of an intersection of the first pipeline with the second pipeline and connected to the inlet end of the cell lysing reactor for mixing feed liquids in respective pipelines; and the second static mixer is disposed in a pipeline downstream of an intersection of the third pipeline with the outlet end of the cell lysing reactor and connected to the inlet end of the neutralization and stationary incubation tank for mixing feed liquids in respective pipelines.

In a specific example, the second pipeline includes: an alkaline solution conveying pipeline, a surfactant conveying pipeline, and an alkaline lysate pipeline, where an inlet end of the alkaline lysate pipeline is connected to the alkaline solution conveying pipeline and the surfactant conveying pipeline, and an outlet end of the alkaline lysate pipeline intersects with an outlet end of the first pipeline; or an alkaline lysate pipeline, the outlet end of the alkaline lysate pipeline intersecting with the outlet end of the first pipeline.

In a specific example, a first valve, a first pump and a first measurement unit are provided in the first pipeline, the first measurement unit including a first flowmeter and a first pressure detector.

In a specific example, the first pipeline is connected to a first purified water port and a second purified water port.

In a specific example, a second valve, a second pump and a second measurement unit are provided in the second pipeline, the second measurement unit including a second flowmeter and a second pressure detector.

In a specific example, the second pipeline is connected to a third purified water port.

In a specific example, a third valve, a third pump and a third measurement unit are provided in the third pipeline, the third measurement unit including a third flowmeter and a third pressure detector.

In a specific example, the third pipeline is connected to a fourth purified water port.

In a specific example, the alkaline lysis device further includes a cleaning pipeline, the cleaning pipeline being in communication with the cell lysing reactor, the neutralization and stationary incubation tank, the first mixer, the second mixer, and various pipelines of the alkaline lysis device.

In a specific example, a fourth valve, a fourth pump and a fourth measurement unit are provided in the cleaning pipeline, the fourth measurement unit including a third conductivity detector.

In a specific example, a first spraying portion is provided in the neutralization and stationary incubation tank at a top, and the first spraying portion is connected to the cleaning pipeline and a fifth purified water port.

In a specific example, an explosion ring and/or a stirring means are provided in the neutralization and stationary incubation tank at a bottom.

In a specific example, a jacket is provided outside the neutralization and stationary incubation tank, a liquid is provided in the jacket, the jacket is connected to an electrical heating circulation unit for heating the liquid, and a temperature transmitter is provided inside the neutralization and stationary incubation tank.

In a specific example, the electrical heating circulation unit includes: a tank, where an electrical heating part is provided inside the tank, and the tank is in communication with the liquid in the jacket through a circulation circuit.

In a specific example, a solution in the circulation circuit and the liquid in the jacket are both purified water, the circulation circuit is connected to a sixth purified water port, and a fifth pump is provided in the circulation circuit between a bottom of the tank and a bottom of the jacket.

In a specific example, a discharge port is provided at the bottom of the neutralization and stationary incubation tank, and the discharge port is connected to a first clarifying filter through a fourth pipeline and a depth filtration pipeline.

In a specific example, the first clarifying filter includes: a primary clarifying filter connected to the discharge port, and a secondary clarifying filter connected to the primary clarifying filter, a sixth pump being provided in the fourth pipeline.

In a specific example, the alkaline lysis device includes a water storage tank, an inlet end of the water storage tank being connected to a water supply system, and the water storage tank being connected to the first pipeline through the second purified water port.

In a specific example, the water storage tank is provided with a high level detector and a low level detector.

In a specific example, a second spraying portion is provided in the water storage tank at a top, and a bottom of the water storage tank is connected to a first waste discharge pipeline.

In a specific example, the water supply system is connected to the sixth purified water port and a seventh purified water port through a fifth pipeline, the seventh purified water port being configured for conveying purified water from the water supply system to the first purified water port, the third purified water port, the fourth purified water port, the fifth purified water port and an eighth purified water port, and the eighth purified water port being connected to the second spraying portion.

In a specific example, a seventh pump and a fifth flowmeter are provided in a pipeline through which the seventh purified water port is connected to the first purified water port, the third purified water port, the fourth purified water port, the fifth purified water port and the eighth purified water port.

In a specific example, the first pipeline is provided with a first waste discharge port, the second pipeline is provided with a second waste discharge port, the third pipeline is provided with a third waste discharge port, a pipeline between an intersection of the third pipeline with the outlet end of the cell lysing reactor and the inlet end of the second static mixer is provided with a fourth waste discharge port, a pipeline between an outlet end of the second static mixer and the neutralization and stationary incubation tank is provided with a fifth waste discharge port, and a sixth waste discharge port is provided at the bottom of the neutralization and stationary incubation tank.

In a specific example, each of the first waste discharge port, the second waste discharge port, the third waste discharge port, the fourth waste discharge port, the fifth waste discharge port and the sixth waste discharge port is connected to the first waste discharge pipeline through a second waste discharge pipeline, and discharging is conducted to a liquid waste collection end through the first waste discharge pipeline.

In a specific example, a mixing valve is provided in a pipeline between the fifth waste discharge port and the neutralization and stationary incubation tank.

In a specific example, an interior of the neutralization and stationary incubation tank is connected to instrument air through a sixth pipeline.

In a specific example, the instrument air is connected to the first pipeline, the second pipeline, the third pipeline, the cleaning pipeline, the fourth pipeline, the circulation circuit and the fifth pipeline.

In a specific example, the first static mixer and the second static mixer each include a helical mixer, a grid mixer and/or an X-mixer.

In a specific example, the cell lysing reactor includes a disposable silicone tube, a pressure-resistant tube, or a stainless steel tube.

A method for extracting plasmids using the alkaline lysis device as described includes the steps of: obtaining a bacterial suspension and connecting the bacterial suspension to the first pipeline; obtaining an alkaline lysate and connecting the alkaline lysate to the second pipeline; obtaining an acid liquid and connecting the acid liquid to the third pipeline; setting a flow rate of the bacterial suspension in the first pipeline, a flow rate of the alkaline lysate in the second pipeline and a flow rate of the acid liquid in the third pipeline based on a flow ratio of the bacterial suspension to the alkaline lysate to the acid liquid; initiating lysis by conveying the bacterial suspension in the first pipeline and the alkaline lysate in the second pipeline together into the cell lysing reactor at respective set flow rates for continuous flow lysis, and conveying the acid liquid in the third pipeline together with a lysed feed liquid into the neutralization and stationary incubation tank at a set flow rate for neutralization and stationary incubation for a certain period of time; clarifying, filtering and concentrating an incubated feed liquid, adding an impurity removal agent and carrying out incubation at a specific temperature for a certain period of time before centrifugation, and taking a centrifuged supernatant to be filtered to obtain a clarified and concentrated feed liquid; and loading the clarified and concentrated feed liquid into a molecular sieve chromatography column, and collecting a first elution peak, so as to obtain target plasmids.

In a specific example, the step of obtaining the alkaline lysate includes: mixing an alkaline solution and a surfactant at a volume ratio of 1 : 1, to obtain the alkaline lysate.

In a specific example, the alkaline solution contains 0.1 to 1 moles of sodium hydroxide, and the surfactant contains 1 to 5% sodium lauryl sulfate.

In a specific example, the flow ratio of the bacterial suspension to the alkaline lysate to the acid liquid is (0.7 - 2) : 2 : 1.5.

In a specific example, the flow rate of the bacterial suspension in the first pipeline ranges from 21 to 45 L/h, the flow rate of the alkaline lysate in the second pipeline ranges from 60 to 90 L/h, and the flow rate of the acid liquid in the third pipeline ranges from 45 to 67.5 L/h.

In a specific example, the cell lysing reactor has a volume ranging from 1.5 to 7.5 L, and the cell lysing reactor has a cell lysis time ranging from 1 to 5 minutes.

In a specific example, time for the stationary incubation ranges from 0.5 to 12 hours.

In a specific example, the step of clarifying, filtering and concentrating the incubated feed liquid, adding the impurity removal agent and carrying out incubation at the specific temperature for the certain period of time before centrifugation, and taking the centrifuged supernatant to be filtered to obtain the clarified and concentrated feed liquid includes: conveying the incubated feed liquid to a first clarifying filter for clarification and filtration, and collecting a clarified and filtered feed liquid; and concentrating the collected clarified and filtered feed liquid and adding an impurity removal agent for incubation at a specific temperature for a certain period of time before centrifugation, and taking a centrifuged supernatant to be filtered by a second clarifying filter to obtain the clarified and concentrated feed liquid.

Compared with the prior art, the present invention provides the following beneficial effects.
1. The alkaline lysis device of the present invention can realize online mixing of the bacterial suspension, the alkaline lysate and the acid liquid at a specific ratio and continuous flow lysis, enables to obtain high-quality plasmids, and is simple, convenient and efficient with good stability and reliability.
2. The alkaline lysis device of the present invention is provided with the first static mixer and the second static mixer, the use of the first static mixer enables the bacterial suspension and the alkaline lysate to be mixed uniformly at a specific ratio and then enter the cell lysing reactor for an alkaline lysis reaction of cells, thereby improving the efficiency, stability and reliability of the alkaline lysis reaction, and the use of the second static mixer enables the acid liquid and the lysed feed liquid to be mixed at a specific ratio for neutralization reaction and then conveyed to the neutralization and stationary incubation tank for neutralization and stationary incubation, thereby improving the efficiency, stability and reliability of the neutralization reaction.
3. The alkaline lysis device of the present invention is provided with the cleaning pipeline such that the cell lysing reactor, the neutralization and stationary incubation tank, the first mixer, the second mixer and the various pipelines of the alkaline lysis device can be automatically cleaned with a cleaning liquid as needed, which is fast, efficient, easy to use and inexpensive.
4. The water supply system of the alkaline lysis device of the present invention is connected to the sixth purified water port and the seventh purified water port and the water storage tank through the fifth pipeline to provide a source of water to the plurality of purified water ports and the water storage tank.
5. The alkaline lysis device of the present invention is provided with a plurality of waste discharge ports, which can facilitate discharging liquid waste to prevent environmental pollution with good safety.
6. The alkaline lysis device of the present invention is connected to the instrument air, which can facilitate the functions of draining liquid by introducing compressed air and drying the device.
7. The alkaline lysis device of the present invention has a simple structure, is easy to use, and has a wide range of applications.
8. The method for extracting plasmids using alkaline lysis device of the present invention enables to obtain high-quality plasmids, and is simple, convenient and efficient.

### Brief Description of the Drawings

FIG. 1 shows a structural schematic view of a specific example of an alkaline lysis device according to the present invention;
FIG. 2 shows a structural schematic view of a specific example of an electrical heating circulation unit of the alkaline lysis device according to the present invention which is connected to a jacket;
FIG. 3 shows a structural schematic view of a specific example of a water supply system of the alkaline lysis device according to the present invention which is connected to a sixth purified water port, a seventh purified water port and a water storage tank through a fifth pipeline;
FIG. 4 shows a structural schematic view of a specific example of a cell lysing reactor of the alkaline lysis device according to the present invention;
FIG. 5 shows a structural schematic view of a specific example of the alkaline lysis device according to the present invention with a first static mixer and a second static mixer being both helical mixers;
FIG. 6 shows a structural schematic view of a specific example of the alkaline lysis device according to the present invention with the first static mixer and the second static mixer being both grid mixers;
FIG. 7 shows a structural schematic view of a specific example of the alkaline lysis device according to the present invention with the first static mixer and the second static mixer being both X-mixers; and
FIG. 8 shows a schematic flow diagram of a specific example of extracting plasmids using an alkaline lysis device according to the present invention.

In the figures: 1-first pipeline; 11-first pump; 12-first measurement unit; 2-second pipeline; 21-second pump; 22-second measurement unit; 3-third pipeline; 31-third pump; 32-third measurement unit; 4-cell lysing reactor; 5-neutralization and stationary incubation tank; 51-first spraying portion; 52-explosion ring; 53-jacket; 54-temperature transmitter; 55-discharge port; 56-fourth pipeline; 57-depth filtration pipeline; 58-sixth pump; 6-first static mixer; 7-second static mixer; 8-cleaning pipeline; 81-fourth pump; 9-electrical heating circulation unit; 91-tank; 92-electrical heating part; 93-circulation circuit; 94-fifth pump; 95-level sensor; 10-water storage tank; 101-high level detector; 102-low level detector; 103-second spraying portion; 13-first waste discharge pipeline; 14-fifth pipeline; 15-seventh pump; 16-fifth flowmeter; 17-second waste discharge pipeline; 18-mixing valve; 19-sixth pipeline.

### Detailed Description of Embodiments

The present invention will be further described below with reference to the examples illustrated in the accompanying drawings.

Directional terms mentioned in the present invention such as "inlet", "outlet", "inner", "outer", "top", "bottom", etc., are only a way to refer to additional drawings. Therefore, the directional terms are used to illustrate and understand the present invention, and are not intended to limit the present invention.

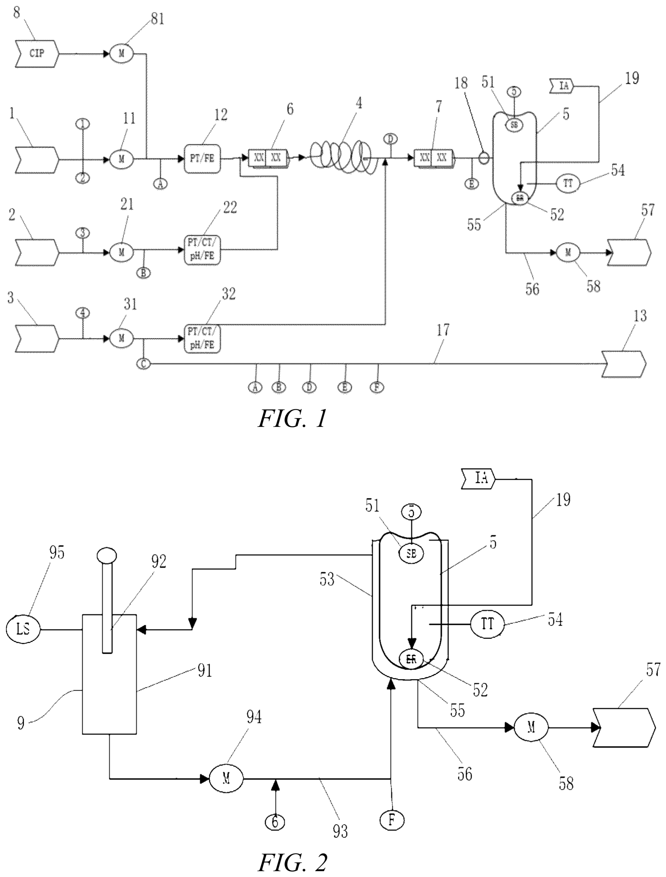

As shown in FIG. 1, an alkaline lysis device according to the present invention includes: a first pipeline 1, a second pipeline 2, a third pipeline 3, a cell lysing reactor 4, and a neutralization and stationary incubation tank 5.

The first pipeline 1 is configured for conveying a bacterial suspension.

The second pipeline 2 is configured for conveying an alkaline lysate.

The third pipeline 3 is configured for conveying an acid liquid.

The cell lysing reactor 4 is used as a reaction vessel for alkaline lysis of cells.

The neutralization and stationary incubation tank 5 is configured for collecting a neutralized feed liquid and carrying out stationary incubation.

The first pipeline 1 and the second pipeline 2 intersect and then are connected to an inlet end of the cell lysing reactor 4. The third pipeline 3 and an outlet end of the cell lysing reactor 4 intersect and then are connected to an inlet end of the neutralization and stationary incubation tank 5.

The bacterial suspension in the first pipeline 1 and the alkaline lysate in the second pipeline 2 are conveyed together into the cell lysing reactor 4 at respective set flow rates for continuous flow lysis. The acid liquid in the third pipeline 3 is conveyed into the neutralization and stationary incubation tank 5 together with a lysed feed liquid at a set flow rate for neutralization and stationary incubation for a certain period of time. The device can realize online mixing of the bacterial suspension, the alkaline lysate and the acid liquid at a specific ratio and continuous flow lysis, and is simple, convenient and efficient with good stability and reliability.

In a specific example, as shown in FIG. 1, the alkaline lysis device includes: a first static mixer 6 and a second static mixer 7.

The first static mixer 6 is disposed in a pipeline downstream of an intersection of the first pipeline 1 with the second pipeline 2 and connected to the inlet end of the cell lysing reactor 4 for mixing feed liquids in respective pipelines.

The second static mixer 7 is disposed in a pipeline downstream of an intersection of the third pipeline 3 with the outlet end of the cell lysing reactor 4 and connected to the inlet end of the neutralization and stationary incubation tank 5 for mixing feed liquids in respective pipelines.

The use of the first static mixer 6 enables the bacterial suspension and the alkaline lysate to be mixed uniformly at a specific ratio and then enter the cell lysing reactor 4 for an alkaline lysis reaction of cells, thereby improving the efficiency, stability and reliability of the alkaline lysis reaction. The use of the second static mixer 7 enables the acid liquid and the lysed feed liquid to be mixed at a specific ratio for neutralization reaction and then conveyed into the neutralization and stationary incubation tank 5 for neutralization and stationary incubation, thereby improving the efficiency, stability and reliability of the neutralization reaction.

In a specific example, the second pipeline 2 includes: an alkaline solution conveying pipeline, a surfactant conveying pipeline, and an alkaline lysate pipeline. An inlet end of the alkaline lysate pipeline is connected to the alkaline solution conveying pipeline and the surfactant conveying pipeline, and an outlet end of the alkaline lysate pipeline intersects with an outlet end of the first pipeline 1. The alkaline solution conveying pipeline is configured for conveying an alkaline solution, and the surfactant conveying pipeline is configured for conveying a surfactant. The alkaline solution conveying pipeline and the surfactant conveying pipeline are connected to the inlet end of the alkaline lysate pipeline to facilitate the alkaline solution and the surfactant being mixed uniformly at a target volume ratio, and an alkaline lysate reaching the target ratio is output through the alkaline lysate pipeline, which is convenient and efficient with good stability and reliability. In another specific example, the second pipeline 2 may be a pipeline which conveying an alkaline lysate, and the outlet end of the alkaline lysate pipeline intersects with the outlet end of the first pipeline 1. The alkaline lysate pipeline is configured for conveying the alkaline lysate formed in advance by mixing the alkaline solution and the surfactant.

In a specific example, a first valve and a first pump 11 are provided in the first pipeline 1. The first valve is used to facilitate the opening and/or closing of the first pipeline 1. The first pump 11 is used to facilitate controlling a flow rate of the bacterial suspension in the first pipeline 1.

In a specific example, as shown in FIG. 1, a first measurement unit 12 is provided in the first pipeline 1, and the first measurement unit 12 includes a first flowmeter (FE) and a first pressure detector (pressure transducer PT). The first flowmeter is used to facilitate measuring the flow of the bacterial suspension in the first pipeline 1. The first pressure detector is used to facilitate measuring a value of the pressure of the bacterial suspension in the first pipeline 1, and is simple in terms of structure and easy to use.

In a specific example, as shown in FIG. 1, a second valve and a second pump 21 are provided in the second pipeline 2. The second valve is used to facilitate the opening and/or closing of the second pipeline 2. The second pump 21 is used to facilitate controlling a flow rate of the alkaline lysate in the second pipeline 2.

In a specific example, as shown in FIG. 1, a second measurement unit 22 is provided in the second pipeline 2. The second measurement unit 22 includes a second flowmeter and a second pressure detector (pressure transducer PT). The second flowmeter is used to facilitate measuring the flow of the alkaline lysate in the second pipeline 2. The second pressure detector is used to facilitate measuring a value of the pressure of the alkaline lysate in the second pipeline 2. In another specific example, as shown in FIG. 1, the second measurement unit 22 further includes a first conductivity detector (conductivity meter CT) and/or a first acidity meter (pH). The first conductivity detector is used to facilitate measuring the conductivity of the alkaline lysate in the second pipeline 2. The first acidity meter is used to facilitate measuring the pH of the alkaline lysate in the second pipeline 2.

In a specific example, as shown in FIG. 1, a third valve and a third pump 31 are provided in the third pipeline 3. The third valve is used to facilitate the opening and/or closing of the third pipeline 3. The third pump 31 is used to facilitate controlling a flow rate of the acid liquid in the third pipeline 3.

In a specific example, as shown in FIG. 1, a third measurement unit 32 is provided in the third pipeline 3. The third measurement unit 32 includes a third flowmeter and a third pressure detector. The third flowmeter is used to facilitate measuring the flow of the acid liquid in the third pipeline 3. The third pressure detector is used to facilitate measuring a value of the pressure of the acid liquid in the third pipeline 3. In another specific example, as shown in FIG. 1, the third measurement unit 32 further includes a second conductivity detector and/or a second acidity meter. The second conductivity detector is used to facilitate measuring the conductivity of the acid liquid in the third pipeline 3. The second acidity meter is used to facilitate measuring the pH of the acid liquid in the third pipeline 3.

In a specific example, as shown in FIG. 1, the alkaline lysis device further includes a cleaning pipeline 8 (CIP). The cleaning pipeline 8 is connected to the cell lysing reactor 4, the neutralization and stationary incubation tank 5, the first mixer 6, the second mixer 7 and various pipelines of the alkaline lysis device. The cleaning pipeline 8 is in communication with the interior of the neutralization and stationary incubation tank 5 through the first pipeline 1, and can clean the interiors of the first pipeline 1, the pipeline connecting the first pipeline 1 and the neutralization and stationary incubation tank 5, and the neutralization and stationary incubation tank 5. It is also possible to clean the interiors of the first mixer 6, the second mixer 7 and the cell lysing reactor 4. The cleaning pipeline 8 can also clean the second pipeline 2, the third pipeline 3 and/or other pipelines, with the neutralization and stationary incubation tank 5 as an circulation center, by opening respective valves of the second pipeline 2, the third pipeline 3 and/or the other pipelines. The cleaning pipeline 8 can carry out cleaning automatically as needed.

In a specific example, as shown in FIG. 1, the cleaning pipeline 8 includes an alkaline solution cleaning pipeline, which is fast and efficient, has significant effects, is easy to use and is inexpensive. The cleaning pipeline 8 can also convey other cleaning liquids.

In a specific example, as shown in FIG. 1, a fourth valve and a fourth pump 81 are provided in the cleaning pipeline 8. The fourth valve is used to facilitate the opening and/or closing of the cleaning pipeline 8. The fourth pump 81 is used to facilitate controlling a flow rate of the cleaning liquid in the cleaning pipeline 8.

In a specific example, as shown in FIG. 1, a fourth measurement unit is provided in the cleaning pipeline 8. The fourth measurement unit includes a third conductivity detector. The third conductivity detector is used to facilitate measuring the conductivity of the cleaning liquid in the cleaning pipeline 8.

In a specific example, as shown in FIGS. 1-3, a first spraying portion (SB) 51 is provided in the neutralization and stationary incubation tank 5 at a top, and the first spraying portion 51 is connected to the cleaning pipeline 8 to facilitate using the alkaline solution in the cleaning pipeline 8 to clean the interior of the neutralization and stationary incubation tank 5. As an example, the first spraying portion 51 includes a spray ball, which has a good cleaning effect with the alkaline solution and is simple in terms of structure and easy to use.

In a specific example, as shown in FIGS. 1-3, an explosion ring (ER) 52 and/or a stirring means are provided in the neutralization and stationary incubation tank 5 at a bottom, so as to facilitate the floating of sediments in the feed liquid in the neutralization and stationary incubation tank 5.

In a specific example, as shown in FIG. 2, a jacket 53 is provided outside the neutralization and stationary incubation tank 5, and a liquid is provided in the jacket 53. The jacket 53 is connected to an electrical heating circulation unit 9, the electrical heating circulation unit 9 is configured to heat the liquid, and a temperature transmitter (TT) 54 is provided inside the neutralization and stationary incubation tank 5. The electrical heating circulation unit 9 is used to heat the liquid in the heating jacket 53 circularly, which in turn allows for the heating of the cleaning liquid (alkaline solution) in the neutralization and stationary incubation tank 5, thereby improving the cleaning effect with the alkaline solution. The temperature transmitter 54 is used to be able to measure the temperature in the neutralization and stationary incubation tank 5 in real time and provide feedbacks to the electrical heating circulation unit 9 during circular heating. The temperature of the cleaning liquid in the cleaning pipeline 8 is 30~60°C (degrees Celsius), and preferably 50°C.

In a specific example, as shown in FIG. 2, the electrical heating circulation unit 9 includes: a tank 91, where an electrical heating part 92 is provided inside the tank 91, and the tank 91 is in communication with the liquid in the jacket 53 through a circulation circuit 93 to facilitate circularly heating the liquid in the jacket 53, and is simple in terms of structure and easy to use.

In a specific example, as shown in FIG. 2, a solution in the circulation circuit 93 and the liquid in the jacket 53 are both purified water, which facilitates heating and circulation and is economical.

In a specific example, as shown in FIG. 2, a fifth pump 94 is provided in the circulation circuit 93 between a bottom of the tank 91 and a bottom of the jacket 53, which facilitates controlling a flow rate of the solution in the circulation circuit 93 and facilitates the circulation of the solution in the circulation circuit 93.

In a specific example, as shown in FIG. 2, the tank 91 is provided with a level sensor (LS) 95 to facilitate measuring the liquid level within the tank 91.

In a specific example, as shown in FIG. 2, a discharge port 55 is provided at the bottom of the neutralization and stationary incubation tank 5, and the discharge port 55 is connected to a first clarifying filter through a fourth pipeline 56, so as to facilitate the entry of the incubated feed liquid discharged from the neutralization and stationary incubation tank 5 into the first clarifying filter for clarification and filtration. As an example, the fourth pipeline 56 is connected to the first clarifying filter through a depth filtration pipeline 57.

In a specific example, the first clarifying filter includes: a primary clarifying filter connected to the discharge port 55 and a secondary clarifying filter connected to the primary clarifying filter to improve the effect of clarification and filtration.

In a specific example, the primary clarifying filter has a filtration accuracy of 50 µm (microns). The secondary clarifying filter has a filtration accuracy of 0.6 microns.

In a specific example, as shown in FIG. 2, a sixth pump 58 is provided in the fourth pipeline 56 to facilitates conveying the incubated feed liquid through the depth filtration pipeline 57 to the first clarifying filter for clarification and filtration and controlling a flow rate of the incubated feed liquid.

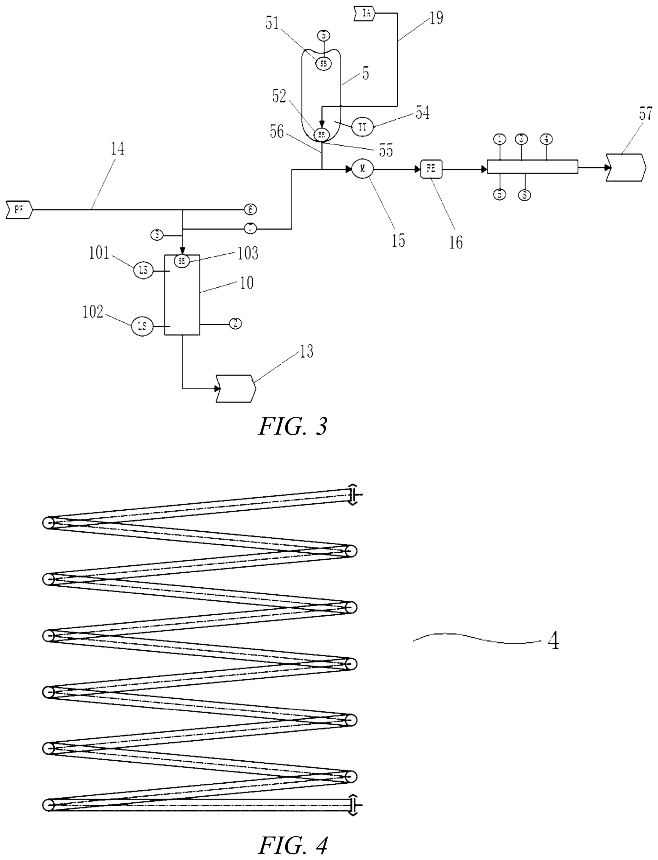

In a specific example, as shown in FIG. 3, the alkaline lysis device includes a water storage tank 10, and an inlet end of the water storage tank 10 is connected to a water supply system (process water PW). The water storage tank 10 is configured for temporary storage of water for the device and can function to provide a stable source of water.

In a specific example, as shown in FIG. 3, the water storage tank 10 is provided with a high level detector 101 and a low level detector 102 to control the level of the water in the water storage tank 10 and automatically replenish the purified water in the water storage tank 10.

In a specific example, as shown in FIG. 3, a second spraying portion 103 is provided in the water storage tank 10 at the top to facilitate flushing and cleaning of the interior of the water storage tank 10. As an example, the second spraying portion 103 includes a spray ball, which has good effects of flushing and cleaning and is simple in terms of structure and easy to use.

In a specific example, as shown in FIG. 3, a bottom of the water storage tank 10 is connected to a first waste discharge pipeline 13 to facilitate discharging waste water from the water storage tank 10 to a liquid waste collection end through the first waste discharge pipeline 13.

In a specific example, as shown in FIGS. 1 and 3, the first pipeline 1 is connected to a first purified water port ① and a second purified water port ②. The first purified water port ① is connected to the water supply system for flushing and cleaning the first pipeline 1. The second purified water port ② is connected to the water storage tank 10. The purified water exiting the second purified water port ② is used to facilitate adjusting the flow of the bacterial suspension in the first pipeline 1 to a target value and placing the flow of the bacterial suspension in the first pipeline 1 in equilibrium. And, after the continuous flow lysis in the device is completed, the purified water exiting the second purified water port ② is used to eject the bacterial suspension in the first pipeline 1.

In a specific example, as shown in FIGS. 1 and 3, the second pipeline 2 is connected to a third purified water port ③. The third purified water port ③ is connected to the water supply system for flushing and cleaning the second pipeline 2.

In a specific example, as shown in FIGS. 1 and 3, the third pipeline 3 is connected to a fourth purified water port ④. The fourth purified water port ④ is connected to the water supply system for flushing and cleaning the third pipeline 3.

In a specific example, as shown in FIGS. 1 and 3, the first spraying portion 51 is connected to a fifth purified water port ⑤. The fifth purified water port ⑤ is connected to the water supply system for flushing and cleaning the neutralization and stationary incubation tank 5.

In a specific example, as shown in FIG. 3, the water supply system is connected to a sixth purified water port ⑥ and a seventh purified water port ⑦ through a fifth pipeline 14. The seventh purified water port ⑦ is configured for conveying the purified water from the water supply system to the first purified water port ①, the third purified water port ③, the fourth purified water port ④, the fifth purified water port ⑤ and an eighth purified water port ⑧. The eighth purified water port ⑧ is connected to the second spraying portion 103 to facilitate flushing and cleaning the water storage tank 10. The sixth purified water port ⑥ is connected to the circulation circuit 93 and can be used to provide water as a circulating solution in the circulation circuit 93.

In a specific example, as shown in FIG. 3, a seventh pump 15 and a fifth flowmeter 16 are provided in a pipeline through which the seventh purified water port ⑦ is connected to the first purified water port ①, the third purified water port ③, the fourth purified water port ④, the fifth purified water port ⑤ and the eighth purified water port ⑧. The seventh pump 15 is used to facilitate conveying the purified water from the water supply system to the first purified water port ①, the third purified water port ③, the fourth purified water port ④, the fifth purified water port ⑤ and the eighth purified water port ⑧. The fifth flowmeter 16 is used to facilitate measuring the flow of the purified water in a corresponding pipeline.

In a specific example, as shown in FIGS. 1 and 2, a first waste discharge port Ⓐ is provided in the first pipeline 1 to discharge liquid waste as needed. A second waste discharge port Ⓑ is provided in the second pipeline 2 to discharge liquid waste as needed. A third waste discharge port Ⓒ is provided in the third pipeline 3 to discharge liquid waste as needed. A fourth waste discharge port Ⓓ is provided in a pipeline between an intersection of the third pipeline 3 with the outlet end of the cell lysing reactor 4 and an inlet end of the second static mixer 7 to discharge liquid waste as needed. A fifth waste discharge port Ⓔ is provided in a pipeline between an outlet end of the second static mixer 7 and the neutralization and stationary incubation tank 5 to discharge liquid waste as needed. A sixth waste discharge port Ⓕ is provided at the bottom of the neutralization and stationary incubation tank 5 to discharge liquid waste as needed.

In a specific example, as shown in FIG. 1, each of the first waste discharge port Ⓐ, the second waste discharge port Ⓑ, the third waste discharge port Ⓒ, the fourth waste discharge port Ⓓ, the fifth waste discharge port Ⓔ and the sixth waste discharge port Ⓕ is connected to the first waste discharge pipeline 13 through a second waste discharge pipeline 17, and discharging is conducted to the liquid waste collection end through the first waste discharge pipeline 13, to prevent environmental pollution with good safety.

In a specific example, a mixing valve 18 is provided in a pipeline between the fifth waste discharge port Ⓔ and the neutralization and stationary incubation tank 5. When the flow rate of the bacterial suspension in the first pipeline 1, the flow rate of the alkaline lysate in the second pipeline 2 and the flow rate of the acid liquid in the third pipeline 3 all reach respective set values, the mixing valve 18 is opened to initiate continuous flow lysis with good controllability.

In a specific example, as shown in FIGS. 1-3, the interior of the neutralization and stationary incubation tank 5 is connected to instrument air (IA) through a sixth pipeline 19, which can be used for the neutralized feed liquid in the neutralization and stationary incubation tank 5. Compressed air is introduced through the sixth pipeline 19 to promote the floating of the sediments in the feed liquid, and the introduction of the compressed air can serve the functions of draining the liquid and drying the neutralization and stationary incubation tank 5.

In a specific example, the instrument air is connected to the first pipeline 1, the second pipeline 2, the third pipeline 3, the cleaning pipeline 4, a pipeline between the intersection of the first pipeline 1 with the second pipeline 2 and the neutralization and stationary incubation tank 5, the fourth pipeline 56, the circulation circuit 93 and the fifth pipeline 14, such that the functions of draining the liquid and drying the first pipeline 1, the second pipeline 2, the third pipeline 3, the cleaning pipeline 4, the pipeline between the intersection of the first pipeline 1 with the second pipeline 2 and the neutralization and stationary incubation tank 5, the fourth pipeline 56, the circulation circuit 93 and the fifth pipeline 14 can be achieved by introducing the compressed air.

In a specific example, as shown in FIGS. 1 and 4, the cell lysing reactor 4 includes a disposable silicone tube, a pressure-resistant tube, or a stainless steel tube, and has a good cell lysis effect.

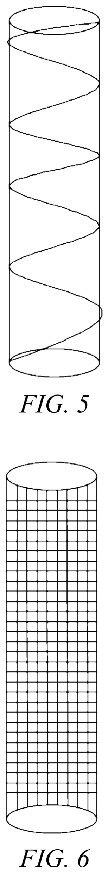

In a specific example, as shown in FIGS. 1 and 5-7, the first static mixer 6 and the second static mixer 7 each include a helical mixer, a grid mixer and/or an X-mixer to improve the effect of uniform mixing of the liquid in the first static mixer 6 and the second static mixer 7 with no damage to the plasmids.

In a specific example, as shown in FIGS. 1-3, the first pump 11, the second pump 21, the third pump 31, the fourth pump 81, the fifth pump 94, the sixth pump 58 and the seventh pump 15 are all membrane pumps that have a good effect of pumping and are explosion-proof and safe with a simple structure and ease of use.

The alkaline lysis device of the present invention can, during use, realize online mixing of the bacterial suspension, the alkaline lysate and the acid liquid at a specific ratio and continuous flow lysis with good stability and reliability. The cleaning pipeline 8 can also be used for automatic cleaning, with good controllability. And, the instrument air can be used to dry and preserve the pipelines.

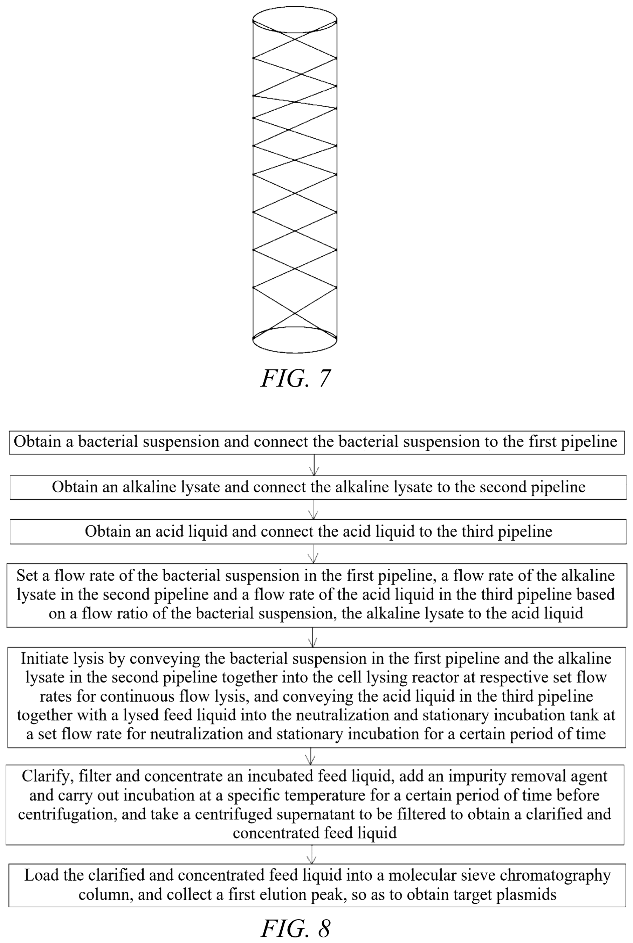

On the basis of the above examples, as shown in FIGS. 1 and 8, the present invention further provides a method for extracting plasmids using the alkaline lysis device as described, the method including the following steps.

At step (1), a bacterial suspension is obtained and is connected to the first pipeline 1. The bacterial suspension is obtained by the resuspension of bacterial cells, and the first pipeline 1 is used to convey the bacterial suspension.

At step (2), an alkaline lysate is obtained and connected to the second pipeline 2. The alkaline lysate can be obtained by mixing an alkaline solution and a surfactant at a specific volume ratio. The second pipeline 2 is used to convey the alkaline lysate.

At step (3), an acid liquid is connected to the third pipeline 3. The acid liquid acts as a neutralization solution. The third pipeline 3 is used to convey the acid liquid.

At step (4), the flow rate of the bacterial suspension in the first pipeline 1, the flow rate of the alkaline lysate in the second pipeline 2 and the flow rate of the acid liquid in the third pipeline 3 are set based on a flow ratio of the bacterial suspension to the alkaline lysate to the acid liquid. The online mixing of the bacterial suspension, the alkaline lysate and the acid liquid at a specific ratio can be achieved by controlling the flow rate of the bacterial suspension in the first pipeline 1, the flow rate of the alkaline lysate in the second pipeline 2 and the flow rate of the acid liquid in the third pipeline 3.

At step (5), lysis is initiated by conveying the bacterial suspension in the first pipeline 1 and the alkaline lysate in the second pipeline 2 together into the cell lysing reactor 4 at respective set flow rates for continuous flow lysis, and the acid liquid in the third pipeline 3 together with the lysed feed liquid is conveyed into the neutralization and stationary incubation tank 5 at the set flow rate for neutralization and stationary incubation for a certain period of time. The flow rate of the bacterial suspension in the first pipeline 1 and the flow rate of the alkaline lysate in the second pipeline 2 can be controlled such that the bacterial suspension and the alkaline lysate in the cell lysing reactor 4 can be sufficiently mixed to improve the effect of online continuous flow lysis, and the effect of online neutralization reaction can be improved by controlling the flow rate of the acid liquid in the third pipeline 3.

At step (6), an incubated feed liquid is conveyed to a first clarifying filter for clarification and filtration, and a clarified and filtered feed liquid is collected. The collection of target plasmids can be facilitated by the clarification and filtration of the incubated feed liquid.

At step (7), the collected clarified and filtered feed liquid is concentrated and added with an impurity removal agent for incubation at a specific temperature for a certain period of time before centrifugation, and a centrifuged supernatant is taken to be filtered by the second clarifying filter to obtain the clarified and concentrated feed liquid. The collection of the target plasmids can be further facilitated by concentrating the clarified and filtered feed liquid.

At step (8), the clarified and concentrated feed liquid is loaded into a molecular sieve chromatography column, and a first elution peak is collected, so as to obtain the target plasmids. The first elution peak is a target plasmid peak.

In a specific example, the step of obtaining the bacterial suspension includes: weighing E. coli cells, adding a bacterial cell resuspension buffer at a certain weight-to-volume ratio, and carrying out stirring with a stirrer for a certain period of time to obtain the bacterial suspension. The content of E. coli plasmids should reach a certain amount.

In a specific example, the weight-to-volume ratio of the E. coli cells to the bacterial cell resuspension buffer ranges from 1 : 5 to 1 : 20, and the stirring time of the stirrer is 0.5~2 hours, so as to obtain a uniform bacterial suspension. Preferably, the stirrer is a magnetic stirrer with a good stirring effect.

In a specific example, the content of the E. coli plasmids is 1-10 mg/g, which can better meet the needs.

In a specific example, the bacterial cell resuspension buffer is formulated with 50 millimoles of glucose, 25 millimoles of tris(hydroxymethyl)aminomethane and 10 millimoles of ethylenediamine tetraacetic acid at a pH of 8.0, which can better meet the needs.

In a specific example, the step of obtaining an alkaline lysate includes: the alkaline solution and the surfactant are mixed at a volume ratio of 1 : 1, to obtain the alkaline lysate. The alkaline lysate has a high quality.

In a specific example, the alkaline solution is formulated with 0.1 to 1 moles of sodium hydroxide, and the surfactant is formulated with 1 to 5% sodium lauryl sulfate, to facilitate obtaining the high-quality alkaline lysate.

In a specific example, the acid liquid is formulated with 3 moles of potassium acetate and 2 moles of acetic acid, which can better meet the needs.

In a specific example, the flow ratio of the bacterial suspension to the alkaline lysate to the acid liquid is (0.7 - 2) : 2 : 1.5, which can better achieve the online mixing of the bacterial suspension, the alkaline lysate and the acid liquid at a specific ratio and improves the efficiencies of the alkaline lysis reaction and the neutralization reaction.

In a specific example, as shown in FIG. 1, the flow rate of the bacterial suspension in the first pipeline 1 ranges from 21 to 45 L/h, the flow rate of the alkaline lysate in the second pipeline 2 ranges from 60 to 90 L/h, and the flow rate of the acid liquid in the third pipeline 3 ranges from 45 to 67.5 L/h, such that the online mixing of the bacterial suspension, the alkaline lysate and the acid liquid at a specific ratio can be further better achieved, and the efficiencies of the alkaline lysis reaction and the neutralization reaction are high.

In a specific example, as shown in FIGS. 1 and 4, the cell lysing reactor 4 has a volume ranging from 1.5 to 7.5 L, and the cell lysing reactor 4 has a cell lysis time ranging from 1 to 5 minutes, such that the alkaline lysis reaction can be sufficient, thereby improving the effect of the alkaline lysis reaction.

In a specific example, time for the stationary incubation ranges from 0.5 to 12 hours to facilitate the subsequent collection of the target plasmids.

In a specific example, the step of concentrating the collected clarified and filtered feed liquid and adding the impurity removal agent for incubation at the specific temperature for the certain period of time before centrifugation, and taking the centrifuged supernatant to be filtered by the second clarifying filter to obtain the clarified and concentrated feed liquid includes: concentrating the collected clarified and filtered feed liquid using a 5-100 kDa membrane cassette, adding an equal volume of 4 moles of ammonium sulfate, carrying out incubation at room temperature for 10-20 minutes before centrifugation with a centrifugal force of 6,000-10,000 gravitational acceleration for 10-20 minutes, and taking centrifuged supernatant to be filtered by the second clarifying filter to obtain the clarified and concentrated feed liquid, which facilitates the subsequent collection of the target plasmids.

In a specific example, for high-copy plasmids, the clarified and filtered feed liquid is concentrated by a factor of 10 or more but less than 50; and for low-copy plasmids, the clarified and filtered feed liquid is concentrated by a factor of 50 or more but less than 100.

In a specific example, the second clarifying filter has a filtration accuracy ranging from 0.2 to 0.65 microns to facilitate obtaining the clarified and concentrated feed liquid.

Some specific examples are listed below.

### Example 1

At step S1, 808 g (gram) of E. coli cells are weighed, the content of the E. coli plasmids is 4.21 mg/g (milligrams per gram), and a bacterial cell resuspension buffer is added thereto at a weight-to-volume ratio of 1 : 10 g/mL (grams per milliliter), the bacterial cell resuspension buffer is formulated with 50 mM (millimole) Glucose, 25 mM Tris (tris(hydroxymethyl)aminomethane) and 10 mM EDTA (ethylenediamine tetraacetic acid) at a pH of 8.0, stirring is carried out with a magnetic stirrer for 0.5 h (hours) to fully form a uniform bacterial suspension, and the obtained bacterial suspension is connected to the first pipeline 1.

At step S2, an alkaline solution and a surfactant are mixed at a volume ratio of 1 : 1 to obtain an alkaline lysate, and the alkaline lysate is connected to the second pipeline 2. The alkaline solution is formulated with 400 mM NaOH (sodium hydroxide) and the surfactant is formulated with 2% SDS (sodium lauryl sulfate).

At step S3, an acid liquid is connected to the third pipeline 3. The acid liquid is formulated with 3 M (mole) CH3COOK (potassium acetate) and 2 M CH3COOH (acetic acid).

At step S4, the flow rate of the bacterial suspension in the first pipeline 1 is set to be 30 L/h (liters/hour), the flow rate of the alkaline lysate in the second pipeline 2 is set to be 60 L/h, and the flow rate of the acid liquid in the third pipeline 3 is set to be 45 L/h based on a flow ratio of the bacterial suspension to the alkaline lysate to the acid liquid of 1 : 2 : 1.5.

At step S5, lysis is initiated by conveying the bacterial suspension in the first pipeline 1, the alkaline lysate in the second pipeline 2 together into the cell lysing reactor 4 at respective set flow rates for continuous flow lysis, and the acid liquid in the third pipeline 3 together with the lysed feed liquid is conveyed into the neutralization and stationary incubation tank 5 at the set flow rate, a neutralization reaction occurs as soon as the acid liquid comes into contact with the lysed feed liquid, and then neutralization and stationary incubation are carried out in the neutralization and stationary incubation tank 5 for 1 hour. The volume of the cell lysing reactor 4 is set to be 1.5 L, with the cell lysis time of 1 minute.

At step S6, the incubated feed liquid in the neutralization and stationary incubation tank 5 is conveyed to the first clarifying filter through the discharge port 55 for clarification and filtration, and the clarified and filtered feed liquid is collected. The discharge port 55 is connected to a 50 um primary clarifying filter and a 0.6 um secondary clarifying filter, and the incubated feed liquid is filtered by the two clarifying filters and then collected into a disposable liner bag.

At step S7, the clarified and filtered feed liquid is concentrated by a factor of 22 using a 100 kDa (kilodalton) membrane cassette and is then added with an equal volume of 4 M ammonium sulfate for incubation at room temperature for 20 minutes before centrifugation, with centrifugation parameters being 6000 g (gravitational acceleration) and 20 min (minutes), and a centrifuged supernatant is taken to be filtered by a 0.65 um second clarifying filter to obtain a clarified and concentrated feed liquid.

At step S8, the clarified and concentrated feed liquid obtained at step S7 is loaded onto a molecular sieve chromatography column which is filled with Bestarose 6FF from Bestchrom, with a loading flow rate of 60 cm/h (centimeters per hour), a loaded volume of 0.23 CV (molar solution concentration multiplied by volume), and a running flow rate of 120 cm/h (centimeters per hour), and a first elution peak is collected.

### Example 2

At step S1, 612 g of E. coli cells are weighed, the content of the E. coli plasmids is 1.36 mg/g, and a bacterial cell resuspension buffer is added thereto at a weight-to-volume ratio of 1 : 10 g/mL (grams per milliliter), the bacterial cell resuspension buffer is formulated with 50 mM (millimole) Glucose, 25 mM tris(hydroxymethyl)aminomethane and 10 mM ethylenediamine tetraacetic acid at a pH of 8.0, stirring is carried out with a magnetic stirrer for 0.5 hours to fully form a uniform bacterial suspension, and the obtained bacterial suspension is connected to the first pipeline 1.

At step S2, an alkaline solution and a surfactant are mixed at a volume ratio of 1 : 1 to obtain an alkaline lysate, and the alkaline lysate is connected to the second pipeline 2. The alkaline solution is formulated with 400 mM sodium hydroxide and the surfactant is formulated with 2% sodium lauryl sulfate.

At step S3, an acid liquid is connected to the third pipeline 3. The acid liquid is formulated with 3 moles of potassium acetate and 2 moles of acetic acid.

At step S4, the flow rate of the bacterial suspension in the first pipeline 1 is set to be 30 L/h (liters/hour), the flow rate of the alkaline lysate in the second pipeline 2 is set to be 60 L/h, and the flow rate of the acid liquid in the third pipeline 3 is set to be 45 L/h based on a flow ratio of the bacterial suspension to the alkaline lysate to the acid liquid of 1 : 2 : 1.5.

At step S5, lysis is initiated by conveying the bacterial suspension in the first pipeline 1, the alkaline lysate in the second pipeline 2 together into the cell lysing reactor 4 at respective set flow rates for continuous flow lysis, and the acid liquid in the third pipeline 3 together with the lysed feed liquid is conveyed into the neutralization and stationary incubation tank 5 at the set flow rate, a neutralization reaction occurs as soon as the acid liquid comes into contact with the lysed feed liquid, and then neutralization and stationary incubation are carried out in the neutralization and stationary incubation tank 5 for 1 hour. The volume of the cell lysing reactor 4 is set to be 1.5 L, with the cell lysis time of 1 minute.

At step S6, the incubated feed liquid in the neutralization and stationary incubation tank 5 is conveyed to the first clarifying filter through the discharge port 55 for clarification and filtration, and the clarified and filtered feed liquid is collected. The discharge port 55 is connected to a 50 um primary clarifying filter and a 0.6 um secondary clarifying filter, and the incubated feed liquid is filtered by the two clarifying filters and then collected into a disposable liner bag.

At step S7, the clarified and filtered feed liquid is concentrated by a factor of 66 using a 100 kDa (kilodalton) membrane cassette and is then added with an equal volume of 4 M ammonium sulfate for incubation at room temperature for 20 minutes before centrifugation, with centrifugation parameters being 6000 g (gravitational acceleration) and 20 min (minutes), and a centrifuged supernatant is taken to be filtered by a 0.65 um second clarifying filter to obtain a clarified and concentrated feed liquid.

At step S8, the clarified and concentrated feed liquid obtained at step S7 is loaded onto a molecular sieve chromatography column which is filled with Bestarose 6FF from Bestchrom, with a loading flow rate of 60 cm/h, a loaded volume of 0.23 CV, and a running flow rate of 120 cm/h, and a first elution peak is collected.

The scope of protection of the present invention is not limited to the above-mentioned examples, and it will be apparent to those skilled in the art that various modifications and variations may be made to the present invention without departing from the scope and spirit of the present invention. It is intended that the present invention encompasses such modifications and variations, provided that such modifications and variations fall within the scope of the claims and their equivalents of the present invention.

## Claims

1. An alkaline lysis device, **characterized by** comprising a first pipeline (1), a second pipeline (2), a third pipeline (3), a cell lysing reactor (4), and a neutralization and stationary incubation tank (5); wherein
the first pipeline (1) is configured for conveying a bacterial suspension;
the second pipeline (2) is configured for conveying an alkaline lysate;
the third pipeline (3) is configured for conveying an acid liquid;
the cell lysing reactor (4) is used as a reaction vessel for alkaline lysis of cells;
the neutralization and stationary incubation tank (5) is configured for collecting a neutralized feed liquid and carrying out stationary incubation;
the first pipeline (1) and the second pipeline (2) intersect and then are connected to an inlet end of the cell lysing reactor (4); and the third pipeline (3) and an outlet end of the cell lysing reactor (4) intersect and then are connected to an inlet end of the neutralization and stationary incubation tank (5).

2. The alkaline lysis device according to claim 1, **characterized by** comprising a first static mixer (6) and a second static mixer (7); wherein
the first static mixer (6) is disposed in a pipeline downstream of an intersection of the first pipeline (1) with the second pipeline (2) and connected to the inlet end of the cell lysing reactor (4) for mixing feed liquids in respective pipelines; and
the second static mixer (7) is disposed in a pipeline downstream of an intersection of the third pipeline (3) with the outlet end of the cell lysing reactor (4) and connected to the inlet end of the neutralization and stationary incubation tank (5) for mixing feed liquids in respective pipelines.

3. The alkaline lysis device according to claim 1 or 2, **characterized in that** the second pipeline (2) comprises: an alkaline solution conveying pipeline, a surfactant conveying pipeline, and an alkaline lysate pipeline, wherein an inlet end of the alkaline lysate pipeline is connected to the alkaline solution conveying pipeline and the surfactant conveying pipeline, and an outlet end of the alkaline lysate pipeline intersects with an outlet end of the first pipeline; or
an alkaline lysate pipeline, the outlet end of the alkaline lysate pipeline intersecting with the outlet end of the first pipeline.

4. The alkaline lysis device according to any one of claims 1-3, **characterized in that** a first valve, a first pump (11) and a first measurement unit (12) are provided in the first pipeline (1), the first measurement unit comprising a first flowmeter and a first pressure detector.

5. The alkaline lysis device according to any one of claims 1-4, **characterized in that** the first pipeline (1) is connected to a first purified water port and a second purified water port.

6. The alkaline lysis device according to any one of claims 1-5, **characterized in that** a second valve, a second pump (21) and a second measurement unit (22) are provided in the second pipeline (2), the second measurement unit (22) comprising a second flowmeter and a second pressure detector.

7. The alkaline lysis device according to any one of claims 1-6, **characterized in that** the second pipeline (2) is connected to a third purified water port.

8. The alkaline lysis device according to any one of claims 1-7, **characterized in that** a third valve, a third pump (31) and a third measurement unit (32) are provided in the third pipeline (3), the third measurement unit (32) comprising a third flowmeter and a third pressure detector.

9. The alkaline lysis device according to any one of claims 1-8, **characterized in that** the third pipeline (3) is connected to a fourth purified water port.

10. The alkaline lysis device according to any one of claims 2-9, **characterized by** further comprising a cleaning pipeline (8), the cleaning pipeline (8) being in communication with the cell lysing reactor (4), the neutralization and stationary incubation tank (5), the first mixer (6), the second mixer (7), and various pipelines of the alkaline lysis device.

11. The alkaline lysis device according to claim 10, **characterized in that** a fourth valve, a fourth pump (81) and a fourth measurement unit are provided in the cleaning pipeline (8), the fourth measurement unit comprising a third conductivity detector.

12. The alkaline lysis device according to any one of claims 1-11, **characterized in that** a first spraying portion (51) is provided in the neutralization and stationary incubation tank (5) at a top, the first spraying portion (51) is connected to the cleaning pipeline (8) and a fifth purified water port, and an explosion ring (52) and/or a stirring means are provided in the neutralization and stationary incubation tank (5) at a bottom.

13. The alkaline lysis device according to claim 12, **characterized in that** a jacket (53) is provided outside the neutralization and stationary incubation tank (5), a liquid is provided in the jacket (53), the jacket (53) is connected to an electrical heating circulation unit (9) for heating the liquid, and a temperature transmitter (54) is provided inside the neutralization and stationary incubation tank (5).

14. The alkaline lysis device according to claim 13, **characterized in that** the electrical heating circulation unit (9) comprises a tank (91), wherein an electrical heating part (92) is provided inside the tank (91), and the tank (91) is in communication with the liquid in the jacket (53) through a circulation circuit (93).

15. The alkaline lysis device according to claim 14, **characterized in that** a solution in the circulation circuit (93) and the liquid in the jacket (53) are both purified water, the circulation circuit (93) is connected to a sixth purified water port, and a fifth pump (94) is provided in the circulation circuit (93) between a bottom of the tank (91) and a bottom of the jacket (53).

16. The alkaline lysis device according to any one of claims 1-15, **characterized in that** a discharge port (55) is provided at the bottom of the neutralization and stationary incubation tank (5), and the discharge port (55) is connected to a first clarifying filter through a fourth pipeline (56) and a depth filtration pipeline (57).

17. The alkaline lysis device according to claim 16, **characterized in that** the first clarifying filter comprises a primary clarifying filter connected to the discharge port (55), and a secondary clarifying filter connected to the primary clarifying filter, a sixth pump (58) being provided in the fourth pipeline (56).

18. The alkaline lysis device according to any one of claims 5-17, **characterized by** comprising a water storage tank (10), an inlet end of the water storage tank (10) being connected to a water supply system, and the water storage tank (10) being connected to the first pipeline (1) through the second purified water port.

19. The alkaline lysis device according to claim 18, **characterized in that** the water storage tank (10) is provided with a high level detector (101) and a low level detector (102).

20. The alkaline lysis device according to claim 18, **characterized in that** a second spraying portion (103) is provided in the water storage tank (10) at a top, and a bottom of the water storage tank (10) is connected to a first waste discharge pipeline (13).

21. The alkaline lysis device according to claim 20, **characterized in that** the water supply system is connected to the sixth purified water port and a seventh purified water port through a fifth pipeline 14, the seventh purified water port being configured for conveying purified water from the water supply system to the first purified water port, the third purified water port, the fourth purified water port, the fifth purified water port and an eighth purified water port, and the eighth purified water port being connected to the second spraying portion (103).

22. The alkaline lysis device according to claim 21, **characterized in that** a seventh pump (15) and a fifth flowmeter (16) are provided in a pipeline through which the seventh purified water port is connected to the first purified water port, the third purified water port, the fourth purified water port, the fifth purified water port and the eighth purified water port.

23. The alkaline lysis device according to claim 20, **characterized in that** the first pipeline (1) is provided with a first waste discharge port, the second pipeline (2) is provided with a second waste discharge port, the third pipeline (3) is provided with a third waste discharge port, a pipeline between an intersection of the third pipeline (3) with the outlet end of the cell lysing reactor (4) and the inlet end of the second static mixer (7) is provided with a fourth waste discharge port, a pipeline between an outlet end of the second static mixer (7) and the neutralization and stationary incubation tank (5) is provided with a fifth waste discharge port, and a sixth waste discharge port is provided at the bottom of the neutralization and stationary incubation tank (5).

24. The alkaline lysis device according to claim 23, **characterized in that** each of the first waste discharge port, the second waste discharge port, the third waste discharge port, the fourth waste discharge port, the fifth waste discharge port and the sixth waste discharge port is connected to the first waste discharge pipeline (13) through a second waste discharge pipeline (17), and discharging is conducted to a liquid waste collection end through the first waste discharge pipeline (13).

25. The alkaline lysis device according to claim 23, **characterized in that** a mixing valve (18) is provided in a pipeline between the fifth waste discharge port and the neutralization and stationary incubation tank (5).

26. The alkaline lysis device according to claim 21, **characterized in that** an interior of the neutralization and stationary incubation tank (5) is connected to instrument air through a sixth pipeline (19).

27. The alkaline lysis device according to claim 26, **characterized in that** the instrument air is connected to the first pipeline (1), the second pipeline (2), the third pipeline (3), the cleaning pipeline (8), the fourth pipeline (56), the circulation circuit (93) and the fifth pipeline (14).

28. The alkaline lysis device according to claim 2, **characterized in that** the first static mixer (6) and the second static mixer (7) each comprise a helical mixer, a grid mixer, and/or an X-mixer.

29. The alkaline lysis device according to claim 1, **characterized in that** the cell lysing reactor (4) comprises a disposable silicone tube, a pressure-resistant tube, or a stainless steel tube.

30. A method for extracting plasmids using the alkaline lysis device according to any one of claims 1-29, **characterized by** comprising the steps of:
obtaining a bacterial suspension and connecting the bacterial suspension to the first pipeline (1);
obtaining an alkaline lysate and connecting the alkaline lysate to the second pipeline (2);
obtaining an acid liquid and connecting the acid liquid to the third pipeline (3);
setting a flow rate of the bacterial suspension in the first pipeline (1), a flow rate of the alkaline lysate in the second pipeline (2) and a flow rate of the acid liquid in the third pipeline (3) based on a flow ratio of the bacterial suspension to the alkaline lysate to the acid liquid;
initiating lysis by conveying the bacterial suspension in the first pipeline and the alkaline lysate in the second pipeline together into the cell lysing reactor at respective set flow rates for continuous flow lysis, and conveying the acid liquid in the third pipeline together with a lysed feed liquid into the neutralization and stationary incubation tank at a set flow rate for neutralization and stationary incubation for a certain period of time;
clarifying, filtering and concentrating an incubated feed liquid, adding an impurity removal agent and carrying out incubation at a specific temperature for a certain period of time before centrifugation, and taking a centrifuged supernatant to be filtered to obtain a clarified and concentrated feed liquid; and
loading the clarified and concentrated feed liquid into a molecular sieve chromatography column, and collecting a first elution peak, so as to obtain target plasmids.

31. The method for extracting plasmids according to claim 30, **characterized in that** the step of obtaining the alkaline lysate comprises mixing an alkaline solution and a surfactant at a volume ratio of 1 : 1, to obtain the alkaline lysate.

32. The method for extracting plasmids according to claim 30, **characterized in that** the alkaline solution contains 0.1 to 1 moles of sodium hydroxide, and the surfactant contains 1 to 5% sodium lauryl sulfate.

33. The method for extracting plasmids according to claim 30, **characterized in that** the flow ratio of the bacterial suspension to the alkaline lysate to the acid liquid is (0.7 - 2) : 2 : 1.5.

34. The method for extracting plasmids according to claim 33, **characterized in that** the flow rate of the bacterial suspension in the first pipeline (1) ranges from 21 to 45 L/h, the flow rate of the alkaline lysate in the second pipeline (2) ranges from 60 to 90 L/h, and the flow rate of the acid liquid in the third pipeline (3) ranges from 45 to 67.5 L/h.

35. The method for extracting plasmids according to claim 30, **characterized in that** the cell lysing reactor (4) has a volume ranging from 1.5 to 7.5 L, the cell lysing reactor (4) has a cell lysis time ranging from 1 to 5 minutes, and time for the stationary incubation ranges from 0.5 to 12 hours.

36. The method for extracting plasmids according to claim 30, **characterized in that** the step of clarifying, filtering and concentrating the incubated feed liquid, adding the impurity removal agent and carrying out incubation at the specific temperature for the certain period of time before centrifugation, and taking the centrifuged supernatant to be filtered to obtain the clarified and concentrated feed liquid comprises: conveying the incubated feed liquid to a first clarifying filter for clarification and filtration, and collecting a clarified and filtered feed liquid; and concentrating the collected clarified and filtered feed liquid and adding an impurity removal agent for incubation at a specific temperature for a certain period of time before centrifugation, and taking a centrifuged supernatant to be filtered by a second clarifying filter to obtain the clarified and concentrated feed liquid.
